# EUROPÄISCHE PATENTANMELDUNG

(11) **EP 1 277 723 A1**
(43) Veröffentlichungstag der Anmeldung: **22.01.2003**
(21) Anmeldenummer: 02014929.0
(22) Anmeldetag: 08.07.2002
(51) Int. Cl.: C07C 39/16, C07C 37/20, C07C 37/16, C07B 37/02

(54) **Verfahren zur Herstellung von Bisphenolen**

(30) Priorität: 18.07.2001 DE 10135012
(71) Anmelder: BAYER AG, 51368 Leverkusen (DE)
(72) Erfinder: Van Osselaer, Tony, Dr., 47800 Krefeld (DE); Verhoeven, Werner, Dr., 2920 Kalmthout (BE); Sluyts, Domien, 2490 Hoevenen (BE)

(57) **Zusammenfassung**

In einem Verfahren zur Herstellung von Bisphenolen ausgehend von Phenolen und substituierten Phenolen und Ketonen oder Diolen in Gegenwart von Chlorwasserstoff und einer flüchtigen Schwefelverbindung mit einer SH-Bindung bringt man die Reaktanden in einem Reaktor zur Reaktion, überführt das Gemisch aus Produkt und Ausgangsstoffen in eine Destillationseinrichtung und trennt die Katalysatoren mit dem Reaktionswasser vom Produkt destillativ, wobei wesentlich höhere Umsetzgeschwindigkeiten und Selektivitäten erzielt werden.

## Beschreibung

Die Erfindung betrifft ein Verfahren zur Herstellung von Bisphenolen ausgehend von Phenolen oder substituierten Phenolen und Ketonen oder Diolen in Gegenwart einer starken, flüchtigen Säure, insbesondere Chlorwasserstoff, und einer flüchtigen Schwefelverbindung mit einer SH-Bindung.

Bisphenole sind Rohstoffe für die Herstellung von Polykondensationswerkstoffen wie Epoxidformmassen, Polyethersulfonen, Polyetherketonen oder Polycarbonaten. Die Herstellung von Bisphenolen erfolgt im allgemeinen dadurch, dass Phenol oder dessen substituierte Derivate unter Säurekatalyse und Abspaltung von Wasser mit geeigneten Ketonen zur Reaktion gebracht werden. Das technisch bedeutsamste Bisphenol ist Bisphenol A (BPA), hergestellt aus Phenol und Aceton. Von großer Bedeutung für die Herstellung von Polycarbonaten mit hoher Wärmeformbeständigkeit sind auch Bisphenole, die aus cyclischen Alkanen abgeleitet sind, beispielsweise das Kondensationsprodukt von Phenol und 3,3,5-Trimethylcyclohexanon (BP-TMC).

Als Katalysatoren für die Herstellung von Bisphenolen werden homogen gelöste Säuren wie Chlorwasserstoff oder heterogene saure Festbettkatalysatoren wie sulfonierte vernetzte Polystyrolharze (saure Ionentauscher) eingesetzt. Während die Benutzung von heterogenen Katalysatoren unter bestimmten Gesichtspunkten der Benutzung von homogenen Katalysatoren vorzuziehen ist, ist der EP-A 995 737 zu entnehmen, dass mit diesem Katalysatortyp für bestimmte Ketone kein ausreichender Umsatz und keine ausreichende Selektivität erzielt werden. Daher wird für eine Vielzahl von Ketonen, insbesondere cyclische Ketone, der Einsatz starker Säuren wie Salzsäure als Katalysator bevorzugt. Zur weiteren Steigerung des Umsatzes an Keton und zur Erhöhung der Selektivität der Reaktion werden schwefelhaltige organische Verbindungen wie Alkylmercaptane, Thiocarbonsäuren oder Dialkylsulfide, wie in der US-A 5 210 328 beschrieben, als Cokatalysatoren eingesetzt. Der US-A 5 336 812 ist die Verwendung spezifischer Alkanthiole zu entnehmen, während die EP-A 995 727 die Verwendung von Alkylmercaptanen mit 1 bis 12 Kohlenstoffatomen vorschlägt.

Als Ergebnis der Umsetzung von Phenolen und Ketonen unter den zuvor genannten Bedingungen werden im allgemeinen Mischungen erhalten, die neben nicht umgesetzten Rohstoffen und Wasser das gewünschte Bisphenol, Isomere, Vor- und Folgeprodukte des gewünschten Produkts sowie den eingesetzten Katalysator und Cokatalysator und gegebenenfalls deren Reaktionsprodukte mit den Komponenten des Reaktionssystems enthalten. Um Bisphenolprodukte geeigneter Qualität zur Herstellung hochwertiger Polymerwerkstoffe zu erhalten, ist es notwendig, diese Nebenprodukte und Reaktionskomponenten möglichst vollständig vom Reaktionsprodukt Bisphenol abzutrennen. Hierzu wird gewöhnlich eine Kombination verschiedener Standardreinigungsoperationen wie Kristallisation, Extraktion oder Destillation vorgenommen. Dabei können verschiedene Probleme auftreten. So sind die erhaltenen Bisphenole im allgemeinen thermisch labil, insbesondere in Anwesenheit von katalytisch aktiven Verbindungen, Säuren oder Basen. Besonders problematisch ist dies beim Einsatz homogen verteilter Säuren als Katalysatoren, die in der Produktmischung verbleiben. Nachteilig an der in der EP-A 995 737 beschriebenen Neutralisation mit Basen oder der in der EP-A 679 151 vorgeschlagenen Extraktion der Säuren durch Zugabe von Wasser ist, dass durch diese Maßnahmen große Mengen organisch belasteten Abfallwassers erzeugt werden, die in aufwendigen Reinigungsoperationen aufgearbeitet werden müssen. Weiterhin ist es bei einem derartigen Vorgehen schwierig, die Reaktion unter Abtrennung des Katalysators kontinuierlich durchzuführen. Außerdem ist bei einem derartigen Vorgehen nicht sichergestellt, dass auch der verwendete schwefelhaltige Cokatalysator weitgehend aus dem Reaktionsgemisch abgetrennt wird. Rückstände des Cokatalysators im gereinigten Bisphenol wirken sich negativ auf dessen Eignung zur Herstellung hochwertiger Polykondensationswerkstoffe aus.

Ein weiteres Problem bei der Herstellung von Bisphenolen aus Phenolen und insbesondere aus Ketonen mit mehr als 5 Kohlenstoffatomen besteht darin, dass die Reaktionsmischungen bei hohen Anteilen an produziertem Bisphenol durch Kristallisation des Produkts fest werden können, so dass eine effiziente kontinuierliche Reaktionsführung und Abtrennung des Katalysators nicht mehr möglich ist. Ein anschließendes Aufschmelzen der Reaktionsmischung oder ein Durchführen der Reaktion bei höheren Temperaturen zur Umgehung dieses Problems führt zu unerwünschten Nebenreaktionen und verringerten Selektivitäten. Auch die Reaktionsführung mit hohem Phenolüberschuss oder unvollständigem Umsatz an Keton zur Vermeidung einer hohen Produktkonzentration an Bisphenolen ist nachteilig, weil dadurch die Raumzeitausbeute verringert wird. Außerdem müssen überschüssiges Phenol und Keton im Laufe der Aufarbeitung aus dem Reaktionsprodukt abgetrennt werden. In der EP-A 995 737 wird vorgeschlagen, zunächst Phenol und Keton in einer Vorreaktion bis zu einem Umsatz von mindestens 90 mol-% des Ketons reagieren zu lassen und anschließend eine weitere Menge Phenol und/oder aromatischen Kohlenwasserstoff der Reaktionsmischung zuzusetzen. Ein derartiges Vorgehen ist umständlich, löst nicht das Problem der Abtrennung des Katalysators und führt gegebenenfalls sogar einen weiteren Stoff in das Verfahren ein, der später ebenfalls abgetrennt werden muss.

Die der Erfindung zugrunde liegende Aufgabe besteht darin, ein Verfahren zur Herstellung von Bisphenolen unter Säurekatalyse in Gegenwart eines schwefelhaltigen Cokatalysators bereitzustellen, dass eine hohe Raumzeitausbeute und eine hohe Selektivität aufweist und ein Produkt liefert, das ohne weitere aufwendige Aufarbeitungsschritte der weiteren Reinigung zugeführt werden kann.

Gelöst wird diese Aufgabe durch ein Verfahren zur Herstellung von Bisphenolen, ausgehend von Phenolen oder substituierten Phenolen und Ketonen oder Diolen in Gegenwart von Chlorwasserstoff und einer flüchtigen Schwefelverbindung mit einer SH-Bindung, in dem man die Reaktanden zur Reaktion bringt und anschließend das Bisphenol von nicht umgesetzten Ausgangsstoffen und Katalysatoren durch Destillation trennt.

Im Gegensatz zu den bekannten Verfahren zur Herstellung von Bisphenol erfolgt erfindungsgemäß keine Neutralisation. Darüber hinaus ist die Abtrennung des Produkts durch Destillation in Verfahren zur Erzeugung von Bisphenolen unbekannt. Unmittelbar nach der Reaktion kann durch Zugabe von Wasser die Reaktionsgeschwindigkeit weitgehend stillgelegt und nachher alle flüchtigen Komponenten wie Katalysator, Cokatalysator, Wasser und je nach Flüchtigkeit der Rohstoffe auch diese destillativ von dem Reaktionsgemisch getrennt werden. Diese werden gleichzeitig als Lösungsmittel benutzt und im Überschuss eingesetzt. Die Umsetzungsgeschwindigkeit ist viel höher als bei bekannten Verfahren. Das erfindungsgemäße Verfahren liefert eine hohe Selektivität bei hohen Raumzeitausbeuten. Katalysator und Cokatalysator können von dem Reaktionsprodukt weitgehend abgetrennt werden, ohne dass Spaltungs- oder Umlagerungsreaktionen in nennenswertem Ausmaß auftreten. Durch Isolierung und Rückführung von nicht umgesetztem Keton kann die Reaktion bei teilweisem Umsatz von Keton mit hoher Selektivität durchgeführt werden, ohne dass das Reaktionsgemisch fest wird, verdünnt werden muss oder Verlust an Keton auftritt.

Ausgangsstoffe im erfindungsgemäßen Verfahren sind Phenol und eine Vielzahl von Phenolabkömmlingen ohne Elektronen ziehende Substituenten und mit nicht substituierter 2- und/oder 4-Position. Geeignete Phenolabkömmlinge sind beispielsweise 2-Alkylphenylole, wie o-Cresol, 2-Ethylphenol, 2-Isopropylphenol oder 2-tert.-Butylphenol, 2,6-Dialkylphenole, wie 2,6-Xylinol, 2,6-Diethylphenol, 2-Methyl-6-i-Propylphenol, 2-Methyl-6-tert.-Butylphenol, 2,6-Di-i-Propylphenol, 2,6-Di-tert.-Butylphenol und 2,4-Dialkylphenole, wie 2,4-Xylenol. Besonders bevorzugt werden Phenol oder o-Cresol eingesetzt. Diese Verbindungen sind einerseits Ausgangsmaterial und andererseits Lösungsmittel für das Reaktionsgemisch.

Die mit den zuvor genannten Ausgangsstoffen umzusetzenden weiteren Ausgangsstoffe des erfindungsgemäßen Verfahrens sind Ketone oder Diole. Diese Keton- oder Diolkomponenten können cyclische oder acyclische aliphatische oder aromatischaliphatische Ketonverbindungen sein. Geeignet sind beispielsweise Aceton, Butanon, 2-Pentanon, 3-Pentanon, Cyclopentanon, 3-Alkylcyclopentanon mit einem Alkylrest mit 1 bis 12 Kohlenstoffatomen, 3,3-Dialkylcyclopentanon mit einem Alkylrest mit 1 bis 12 Kohlenstoffatomen, wobei die Alkylreste gleich oder verschieden sein können, 3,3,5-Trialkylcyclohexanon, wobei die Alkylreste 1 bis 12 Kohlenstoffatome aufweisen und gleich oder verschieden sein können, Cyclohexanon, 3-Alkylcyclohexanon mit einem Alkylrest, der 1 bis 12 Kohlenstoffatome aufweist, 4-Alkylcylcohexanon mit einem Alkylrest, der 1 bis 12 Kohlenstoffatome aufweist, 3,3-Dialkylcyclohexanon mit Alkylresten, die 1 bis 12 Kohlenstoffatome aufweisen, wobei die Alkylreste gleich oder verschieden sein können, 3,3,5-Trialkylcyclohexanon mit Alkylresten, die 1 bis 12 Kohlenstoffatome aufweisen und gleich oder verschieden sein können, Acetophenon. Besonders bevorzugt sind mit Alkylresten substituierte Cyclohexanone, deren Alkylrest 1 bis 5 Kohlenstoffatome aufweist. Die Keton- oder Diolkomponenten können in einer Konzentration von 1 bis 25 Gew.%, vorzugsweise 1 bis 20 Gew.%, bezogen auf das Gewicht des Reaktionsgemischs eingesetzt werden.

Geeignete Katalysatoren sind starke flüchtige Säuren, wie konzentrierte Salzsäuren und Chlorwasserstoffgas. Bevorzugt sind konzentrierte Salzsäure, Chlorwasserstoffgas Bromwasserstoffsäure und Trifluoressigsäure. Als Cokatalysator werden flüchtige Schwefelverbindungen mit einer SH-Bindung eingesetzt. Solche Verbindungen umfassen Schwefelwasserstoff, Methyl-, Ethyl- und Propylmercaptan. Bevorzugt ist Schwefelwasserstoff. Besonders bevorzugt wird das erfindungsgemäße Verfahren mit Chlorwasserstoffgas und Schwefelwasserstoff als Katalysator und Cokatalysator durchgeführt. Die Konzentration des Katalysators kann 0,3 bis 5 Gew.-%, vorzugsweise 0,5 bis 2 Gew.-%, bezogen auf die Masse des Reaktionsgemischs betragen. Die Konzentration des Cokatalysators kann 50 ppm bis 1 Gew.-%, vorzugsweise 100 ppm bis 0,5 Gew.-% betragen, bezogen auf die Masse des Reaktionsgemischs was Gewichtsprozent anbetrifft.

Zu Beginn des Verfahrens können die Ausgangsstoffe zusammen mit konzentrierter Säure im Reaktor vorgelegt werden. Unter Bewegen, vorzugsweise Rühren, des Reaktionsgemischs werden Katalysator und Cokatalysator eingeblasen. Der Cokatalysator kann auch in situ erzeugt werden. Dazu kann Ammoniumbisulfid in das saure Reaktionsgemisch eingeleitet werden.

Die Umsetzung kann bei Atmosphärendruck oder bei Überdruck erfolgen. Vorzugsweise wird die Reaktion bei einem Überdruck von 0,1 bis 0,5 MPa (1 bis 5 bar) durchgeführt. Die Temperatur des Reaktionsgemischs kann 10 bis 80°C, vorzugsweise 25 bis 60°C und besonders bevorzugt 30 bis 40°C, betragen.

Während der Reaktion wird Wasser gebildet. Dieses Reaktionsprodukt bremst die Reaktionsgeschwindigkeit, zu deren Aufrechterhalten Katalysator ständig nachgeführt werden muss. Dies gilt auch für den Cokatalysator. Katalysator und Cokatalysator können einzeln oder im Gemisch flüssig, fest oder gasförmig in das Reaktionsgemisch gegeben werden. Vorzugsweise erfolgt die Zugabe gasförmig durch Einblasen in das Reaktionsgemisch. Das Reaktionsgemisch ist im Hinblick auf Katalysator und Cokatalysator vorzugsweise gesättigt. Das erfindungsgemäße Verfahren kann kontinuierlich oder diskontinuierlich, vorzugsweise kontinuierlich, durchgeführt werden.

Aus dem unteren Teil des Reaktors kann die Reaktionslösung ausgeschleust und entweder direkt oder über eine Vorlage in eine Destillationseinrichtung gefördert werden. Während der Destillation werden die Katalysatoren, Wasser sowie nicht umgesetzte Ausgangsmaterialien von der Reaktionslösung getrennt.

Damit Wasser möglichst vollständig bei nicht all zu hohen Temperaturen abgedampft werden kann, erfolgt die Destillation unter Vakuum und das Vakuum kann derart eingestellt werden, dass die Bodentemperatur nicht zur Produktschädigung führt. Dies wird vorzugsweise bei einer Temperatur von 130°C und weniger der Fall sein.

Bei etwa 130°C sind die Bisphenole gelöst und von den Ausgangsverbindungen wie Phenolen und Kresolen wird nur soviel abgedampft, dass eine homogene Lösung bestehen bleibt. Diese Verfahrensweise erlaubt die nahezu vollständige Entfernung des Wassers und der Katalysatoren und Cokatalysatoren und liefert eine saubere konzentrierte Produktlösung, die beim Abkühlen sauber und wirtschaftlich kristallisiert werden kann. Der Druck in der Destillationskolonne beträgt 50 bis 120 mbar, vorzugsweise 90 bis 110 mbar.

Das Bodenprodukt der Kolonne enthält neben dem Bisphenolprodukt noch Phenol und gegebenenfalls Keton, je nach Siedepunkt des Produkts 0 bis 15 Gew.-%, bezogen auf das Gewicht der Lösung. Das Bodenprodukt kann eine Temperatur von 100 bis 130°C aufweisen und wird bei dieser Temperatur in einen üblichen Kristaller überführt. Die Kristallisation erfolgt vorzugsweise in einem Drehkristaller. Je nach erhaltenem Produkt kann ein- bis dreimal, vorzugsweise ein- bis zweimal, umkristallisiert werden. So reicht bei TMC-Phenolen im allgemeinen eine zweimalige und bei BPA im allgemeinen eine einmalige Kristallisation aus, um ein hochreines Produkt zu erhalten. Das Umkristallisieren erfolgt vorzugsweise mit Phenol. Dadurch werden schwer aufzutrennende Mischungen zwischen Phenol und anderen Lösungsmitteln vermieden.

Das kristallisierte Produkt kann anschließend noch von Phenol gereinigt werden. Die Reinigung kann beispielsweise durch thermische Desorption des Phenols bei erhöhten Temperaturen unter Einblasen von Stickstoff erfolgen. Da bei diesem Verfahren Temperaturen von mehr als 190°C erforderlich sind, werden Spaltprodukte gebildet. Gemäß einer besonders bevorzugten Ausführungsform der Erfindung wird deshalb das kristallisierte Produkt mit Wasser gewaschen und dabei Phenol aus den Bisphenol/Phenol-Addukte herausgelöst. Die Temperatur des Wassers zum Waschen des Bisphenols kann 20 bis 100°C, vorzugsweise 70 bis 80°C, betragen. Der abfiltrierte Kuchen kann mit warmen Wasser gewaschen werden. Die Entfernung von Phenol aus den Bisphenol/Phenol-Mischkristallen mit Wasser verhindert eine thermische Schädigung des Bisphenols.

Das erfindungsgemäße Verfahren kann in einem Rührreaktor, einem Umpumpreaktor oder einem Kaskadenreaktor durchgeführt werden. Die Destillation kann eine kontinuierliche oder eine diskontinuierliche Destillation sein. Das Einbringen der Reaktionslösung in die Destillationseinrichtung kann zwischen zwei Trennstufen mit Phenolabnahme bei Totalreflux erfolgen. Vorzugsweise ist der untere Teil der Destillationseinrichtung so ausgerüstet, dass Wasser, Katalysator und Cokatalysator und gegebenenfalls das Ausgangsmaterial mit Phenoldampf abgestreift werden können.

Erfindungsgemäß zu erhaltene Bisphenole (Diphenole) sind vorzugsweise solche der Formel (I) 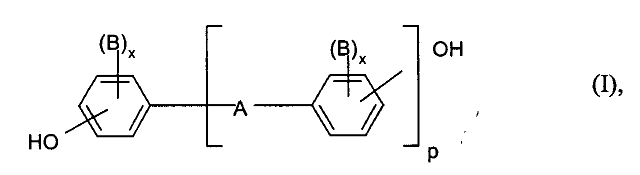 wobei
- A: eine Einfachbindung, C₁ bis C₅-Alkylen, C₂ bis C₅-Alkyliden, C₅ bis C₆-Cycloalkyliden, -O-, -SO-, -CO-, -S-, -SO₂-, C₆ bis C₁₂-Arylen, an das weitere aromatische gegebenenfalls Heteroatome enthaltende Ringe kondensiert sein können,
oder ein Rest der Formel (II) oder (III) 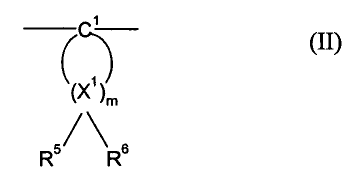 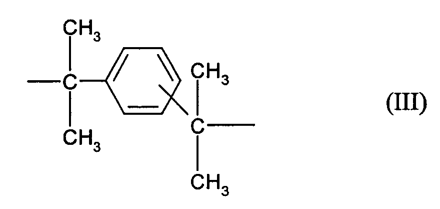
- B: jeweils C₁ bis C₁₂-Alkyl, vorzugsweise Methyl, Halogen, vorzugsweise Chlor und/oder Brom
- x: jeweils unabhängig voneinander 0, 1 oder 2,
- p: 1 oder 0 sind, und
- R⁵ und R⁶: für jedes X¹ individuell wählbar, unabhängig voneinander Wasserstoff oder C₁ bis C₆-Alkyl, vorzugsweise Wasserstoff, Methyl oder Ethyl,
- X¹: Kohlenstoff und
- m: eine ganze Zahl von 4 bis 7, bevorzugt 4 oder 5 bedeuten, mit der Maßgabe, dass an mindestens einem Atom X¹, R⁵ und R⁶ gleichzeitig Alkyl sind.

Bevorzugte Diphenole sind Hydrochinon, Resorcin, Dihydroxydiphenole, Bis-(hydroxyphenyl)-C₁-C₅-alkane, Bis-(hydroxyphenyl)-C₅-C₆-cycloalkane, Bis-(hydroxyphenyl)-ether, Bis-(hydroxyphenyl)-sulfoxide, Bis-(hydroxyphenyl)-ketone, Bis-(hydroxyphenyl)-sulfone und α,α-Bis-(hydroxyphenyl)-diisopropyl-benzole sowie deren kernbromierte und/oder kernchlorierte Derivate.

Besonders bevorzugte Diphenole sind 4,4'-Dihydroxydiphenyl, Bisphenol-A, 2,4-Bis(4-hydroxyphenyl)-2-methylbutan, 1,1-Bis-(4-hydroxyphenyl)-cyclohexan, 1,1-Bis-(4-hydroxyphenyl)-3,3,5-trimethylcyclohexan, 4,4'-Dihydroxydiphenylsulfid, 4,4'-Dihydroxydiphenylsulfon sowie deren di- und tetrabromierten oder chlorierten Derivate wie 2,2-Bis(3-Chlor-4-hydroxyphenyl)-propan, 2,2-Bis-(3,5-dichlor-4-hydroxyphenyl)propan oder 2,2-Bis-(3,5-dibrom-4-hydroxyphenyl)propan.

Durch die folgenden Beispiele wird die Erfindung illustriert, ohne sie jedoch einzuschränken.

### Beispiel 1

In einen Rührreaktor mit einem Volumen von 1 1 werden 500 g Reaktionslösung vorgelegt. Die Reaktionslösung enthält 95,5 Gew.-% Phenol und 4,5 Gew.% Aceton. Zu Beginn der Reaktion wird eine 5 gew.-%ige wässrige HCl zudosiert. Der Gasraum wird unter einem Druck von 2 bar durch HCl-Gas gehalten. Dem Gasstrom werden 3,3 l/h H₂S-Gas zudosiert. Nach etwa 30 Minuten werden in diesen Reaktor 0,8 l/h Reaktionslösung kontinuierlich hineingepumpt, wobei das Verhältnis Phenol/Aceton dem zuvor angegebenen Verhältnis entspricht. Gleichzeitig werden aus dem Reaktor 0,8 l/h Reaktionslösung zur Destillationskolonne gepumpt. Die Steuerung des Volumens im Reaktor erfolgt derart, dass 30 Minuten Verweilzeit erreicht werden. Die Temperatur des Reaktionsgemischs beträgt 40°C.

Die Reaktionslösung wird durch einen Wärmetauscher auf etwa 113°C gebracht. Das Vakuum der Destillationskolonne wird auf 100 mbar eingestellt. Das Refluxverhältnis wird so geregelt, dass nur kleine Mengen Phenol über Kopf abdestillieren. Der Boden der Kolonne wird auf etwa 125°C gefahren. Die flüchtigen Substanzen wie Wasser, Chlorwasserstoff, Schwefelwasserstoff und der Rest des nicht umgesetzten Acetons werden kondensiert und wieder aufgearbeitet.

Der Boden der Destillationskolonne enthält dann nur noch die Reaktionsprodukte und Phenol. Aus der heißen Produktlösung kristallisiert das Addukt Bisphenol/Phenol (45°C). Das Produkt wird abfiltriert und mit warmem Phenol gewaschen. Die Mutterlauge kann recycliert werden. Das gewaschene Addukt wird anschließend mit warmem Wasser bei einer Temperatur von etwa 85°C gewaschen, um Phenol und Bisphenol zu trennen. Nach der Filtration wird das Produkt im Vakuum getrocknet.

Die Selektivität in der Reaktionslösung beträgt 95,5 % p,p-BPA und die Produktkonzentration nach Trocknung 99,71 %.

### Beispiel 2 (Vergleich)

Die Reaktion erfolgt unter den gleichen Reaktionsbedingungen wie in Beispiel 1, wobei jedoch kein H₂S zudosiert wurde. Die Reaktionszeit wurde verdoppelt. Der Acetonumsatz nimmt trotzdem mit 1/3 ab, die Selektivität sinkt aber auf 86,6 % p,p-BPA. Das Vergleichsbeispiel zeigt die Wichtigkeit des Cokatalysators für das erfindungsgemäße Verfahren.

## Patentansprüche

1. Verfahren zur Herstellung von Bisphenolen ausgehend von Phenolen und substituierten Phenolen und Ketonen oder Diolen in Gegenwart von Chlorwasserstoff und einer flüchtigen Schwefelverbindung mit einer SH-Bindung, **dadurch gekennzeichnet, dass** man die Reaktanden in einem Reaktor zur Reaktion bringt, das Gemisch aus Produkt und Ausgangsstoffen in eine Destillationseinrichtung überführt und die Katalysatoren mit dem Reaktionswasser vom Produkt destillativ trennt.

2. Verfahren nach Anspruch 1, **dadurch gekennzeichnet, dass** man die Reaktanden unter der Einwirkung von flüssigen oder gasförmigen Katalysatoren zur Reaktion bringt.

3. Verfahren nach Anspruch 1 oder 2, **dadurch gekennzeichnet, dass** man den gasförmigen Katalysator in situ erzeugt.

4. Verfahren nach einem der Ansprüche 1 bis 3, **dadurch gekennzeichnet, dass** man die Konzentrationen der Salzsäure und/oder der flüchtigen Schwefelverbindung im Reaktionsgemisch konstant bei der Sättigungskonzentration hält.

5. Verfahren nach einem der Ansprüche 1 bis 4, **dadurch gekennzeichnet, dass** man die Reaktionstemperatur in einem Bereich von mindestens 10 bis 80°C hält.

6. Verfahren nach einem der Ansprüche 1 bis 5, **dadurch gekennzeichnet, dass** man die Reaktion unter Druck durchführt.

7. Verfahren nach einem der Ansprüche 1 bis 6, **dadurch gekennzeichnet, dass** man die Temperatur in der Destillationseinrichtung derart einstellt, dass Verluste des Bisphenols minimiert werden.

8. Verfahren nach einem der Ansprüche 1 bis 7, **dadurch gekennzeichnet, dass** man die Temperatur im Sumpf der Destillationseinrichtung derart einstellt, dass das Produkt in Lösung bleibt und/oder geht.

9. Verfahren nach einem der Ansprüche 1 bis 8, **dadurch gekennzeichnet, dass** man die bei der Kristallisation und/oder der Filtration entstehenden Laugen in die Reaktion zurückführt.

10. Verfahren nach einem der Ansprüche 1 bis 9, **dadurch gekennzeichnet, dass** man das Verfahren kontinuierlich durchführt.
